# EUROPEAN PATENT APPLICATION

(11) **EP 3 096 153 A1**
(43) Date of publication of application: **23.11.2016**
(21) Application number: 15168807.4
(22) Date of filing: 22.05.2015
(51) Int. Cl.: G01R 33/48, A61B 6/03, G01R 33/565

(54) **RF COIL WITH DIELECTRIC MATERIAL**

(71) Applicant: MRI.Tools GmbH, 13125 Berlin (DE)
(72) Inventor: Rieger, Jan, 13125 Berlin (DE); Niendorf, Thoralf, 13125 Berlin (DE)

(57) **Abstract**

This invention relates to magnetic resonance imaging (MRI) and spectroscopy (MRS) to single photon emission computed tomography (SPECT) imaging. In particular, the invention relates to the combination of MRI with SPECT systems for the purpose of hybrid imaging and to the use of materials with high dielectric constant or permittivity and with low electrical conductivity for RF field generation and for collimation of ionizing radiation. The invention relates to hybrid imaging and spectroscopy, in particular to industrial, preclinical and clinical applications as well as applications in material sciences.

The invention describes an embodiment where a material is used in proximity of an RF coil. The material has high density and specific electromagnetic parameters (in particular the dielectric constant). The materials function is to collimate and attenuate ionizing radiation and to improve RF field profile of the RF coil.

## Description

This invention relates to magnetic resonance imaging (MRI) and spectroscopy (MRS) to single photon emission computed tomography (SPECT) imaging. In particular, the invention relates to the combination of MRI with SPECT systems for the purpose of hybrid imaging and to the use of materials with high dielectric constant or permittivity and with low electrical conductivity for RF field generation and for collimation of ionizing radiation. The invention relates to hybrid imaging and spectroscopy, in particular to industrial, preclinical and clinical applications as well as applications in material sciences.

The invention describes an embodiment where a material is used in proximity of an RF coil. The material has high density and specific electromagnetic parameters (in particular the dielectric constant). The materials function is to collimate and attenuate ionizing radiation and to improve RF field profile of the RF coil.

### BACKGROUND OF THE INVENTION

The presented invention relates to magnetic resonance imaging (MRI), more particularly, to a design of a radiofrequency (RF) resonator for a hybrid imaging system consisting of MRI and of a single photon emission computed tomography (SPECT) system or another scintillation or a gamma camera system that is combined with the MRI. The purpose of the invention is the generation of a substantially uniform RF electro-magnetic field for the purpose of MRI and the attenuation and collimation of ionizing radiation for the purpose of SPECT imaging.

### MRI

MRI and MRS are non-invasive imaging and spectroscopy methods, based on nuclear magnetic resonance (NMR). The principle of NMR is based on atomic nuclei with an odd number of protons or neutrons. These MR active nuclei possess an intrinsic angular momentum which is always coupled with a magnetic dipole moment. As a result there is a measurable angular momentum and an associated macroscopic magnetic moment that can be disturbed by electro-magnetic fields through the application of radio-frequency (RF) excitation with a frequency centered on the Larmor frequency. The RF excitation is created by an RF coil positioned near the sample so that its field axis is orthogonal to the static magnetic field.

The influence of the applied RF field causing the magnetic flux density *B₁* leads to an angle *α* between the static magnetic field and the net nuclear magnetic moment. This angle is proportional to the duration and power (peak integral) of the RF field. The theory shows, that the magnetization returns to the equilibrium after a finite interval. This process is called free induction decay (FID) or relaxation and can be described by Bloch's equations.

The relaxation of the magnetization can be then detected with the same RF coil which has been used for RF excitation. What makes NMR interesting in the medical diagnostics is that the relaxation times and other (patho)physiology related MR parameters vary for different tissues and thus imaging a biological object produces high levels of contrast for different tissues. This facilitates the different contrast for anatomical or pathological areas.

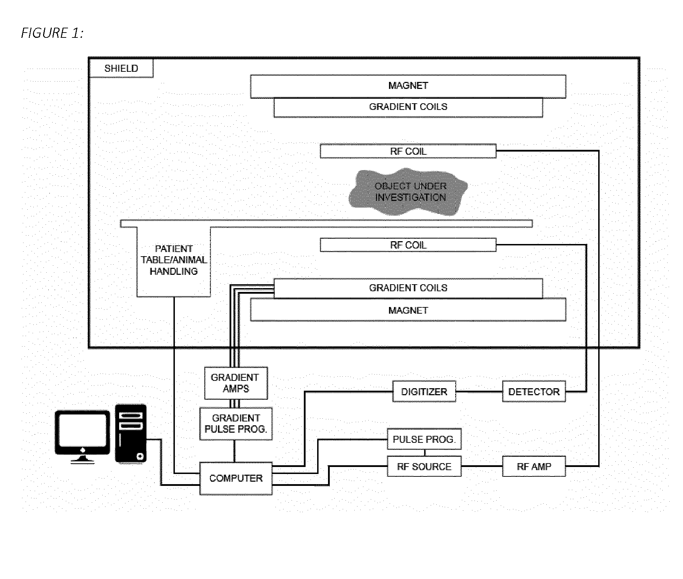

As shown in Fig. 1, an MR imaging system typically includes a magnet that imposes the static magnetic field (B₀), gradient coils for generating spatially distributed gradient magnetic fields (Gx, Gy and Gz) and RF coils to transmit and receive the RF signals to and from the object under investigation. The object under investigation (patient, animal or any arbitrary object) is placed on a patient table or an animal handling bed and moved into the magnet bore which accommodates the RF coil.

### SPECT

In a conventional SPECT imaging study a radioisotope (for example: Tc-99m or In-111 for example) is injected into the object under investigation (patient, animal etc.) and the radioisotope distributes in the object or is taken up by some parts of the object (for example: liver, brain). For detection of the radioisotope the object is placed in a SPECT system. SPECT detectors interact with the gamma radiation from the object and the recorded data is used for reconstruction of two or three-dimensional images of the distribution of the radioisotope in the object.

### THE COLLIMA TOR

In a conventional SPECT imaging study a collimator is used to ensure that only beams of radiation that emanate along a particular path do hit the detector. Herewith, the detection of scattered or secondary radiation beams is minimized. The radiation beams that do not emanate along the desired path are attenuated in the collimator. In a conventional SPECT imaging system the collimator is therefore placed between the object and the detector. Conventionally, the collimator is made of a radiopaque material such as lead and is equipped with holes or apertures.

### HYBRID MRI AND SPECT IMAGING

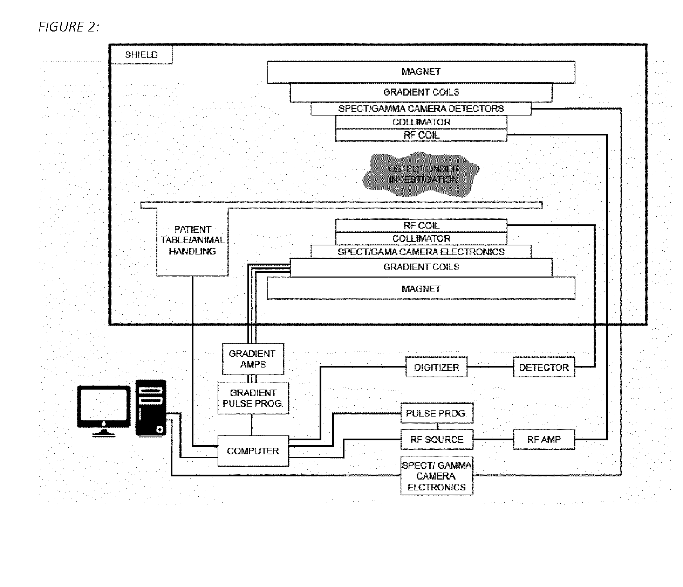

For the purpose of hybrid MR/SPECT imaging a SPECT detector system is inserted into an existing MR system. Alternatively, a new hybrid system is designed that combines the components of an MR and a SPECT system. As illustrated in Fig. 2 the hybrid MR/SPECT system contains the collimator and the detector electronics which are placed between the RF coil and the gradient coil of the MR system. The remaining available space for the RF coil and the object under investigation is therefore limited. The RF coil is therefore closer to the object under investigation and experiences higher loading. Current collimator materials can act as RF shielding. Therefore, from the MRI perspective, it would be desirable to keep the collimator as far away from the RF coils as possible. However, from the SPECT perspective it would be desirable to place the collimator as close to the object under investigation as possible. Hence, there is a need for a solution that will allow for placing the collimator close to the object under investigation and will compensate for the destructive effects caused by the RF shielding that is close to the RF coil.

### DIELECTRIC MATERIALS IN RF COIL

Foo at el. [1] have proposed a method of correcting for the RF inhomogeneity in an RF coil by dielectric loading of the space between the RF coil layout and its RF shield with dielectric material of suitable permittivity so as to alter the propagation constant of the RF coil. In a different work of the state of the art the dielectric material was placed between the RF coil layout and the object under investigation. Thus it was used to couple the RF field produced by the RF coil with the sample. In the current invention both effects are used in order to modulate the RF field and also the material is designed in such a way that it serves as a collimator for the SPECT imaging.

### SUMARY OF THE INVENTION

The features of this invention are defined by the independent claims. Preferred embodiments of the present invention are provided by the dependent claims.

In a first aspect, the invention relates to a device, that is either connected to an existing magnetic resonance system (MR system) or is a part of a dedicated system for the purpose of hybrid application of single photon emission computed tomography and magnetic resonance imaging, comprising an RF coil which generates B₁ magnetic field; and a substrate from a high dielectric material that acts as a collimator of ionizing radiation and couples the RF field produced by the RF coil with the sample.

In the proposed invention the RF coil can be a volume coil (e.g. a linearly polarized birdcage coil or a circularly polarized birdcage coil). It can also be a surface RF coil (e.g. a loop element, a (micro)stripline, a TEM element, a dipole or combinations of the aforementioned building blocks etc.) or it can be a 1D, 2D or 3D RF coil array of RF elements. The RF coil has always an inner side that might be oriented towards the object under investigation and an outer side that might be oriented away from the object under investigation.

At the inner side or at the outer side of the RF coil, or on both sides of the RF coil a substrate material is placed that has such physical parameters (e.g. high density, high dielectric constant) that it both acts as a collimator for the ionizing radiation and it influences the shape of the B₁ field that is generated inside of the object under investigation. The function of a collimator is the important novelty against the state of the art and of the prior art [2]. The effect on the B₁ field is an important novelty against the state of the art and of the prior art [3,4].

In one embodiment of the invention a substrate that includes a high dielectric material (with dielectric constant 30 or higher) is placed at the inner side of the RF coil. This substrate has holes or apertures or another features so that it can act as a collimator of ionizing radiation. At the outer side of the RF coil there is either an RF shield or a collimator from a different material (lead, tungsten etc.) that acts as an RF shield.

The material of the substrate can be manufactured for example from barium titanate (BaTiO₃) or calcium titanate (CaTiO₃). These materials itself don't have sufficient attenuation capability therefore it is necessary to mix them with another compound (e.g. lead or tungsten) or coat the surfaces by these materials.

In another embodiment of the invention a substrate that includes a high dielectric material is placed at the outer side of the RF coil. The substrate has holes or apertures or another features so that it can act as a collimator of ionizing radiation.

In another embodiment of the invention a substrate that includes a high dielectric material is placed at the inner side of the RF coil and at the outer side of the RF coil. The substrate has holes or apertures or another features so that it can act as a collimator of ionizing radiation.

In the proposed embodiments of the invention the substrate can be manufactured from one homogeneous material or it can be manufactured from two or more homogeneous materials. It can be manufactured in a way that different parts of the substrate are manufactured from different materials that have different physical parameters. For example it can be manufactured in a way that a material at the inner side of the RF coil has a relatively low density but a high dielectric constant; simultaneously a material at the outer side of the RF coil might have a high density and lower dielectric constant.

In the proposed embodiments of the invention the substrate can be manufactured from a material that is not homogeneous in its whole volume or it can be manufactured from two or more materials that are not homogenous in its volumes. For example the invention considers a case where the dielectric constant would be low close to the object under investigation and it would increase with the distance from the object under investigation. Similarly, the density of the material could have a certain gradient.

In the embodiments of the proposed invention it is also possible that one part of the substrate is manufactured from a metal (lead, tungsten etc.) or a carbon or a different material or composite that shields the RF signal.

In the embodiments of the proposed invention the substrate can be manufactured from a ceramics, from solid state materials or it can be powder or solved/mixed in slurries. In the embodiments of the proposed invention the substrate can be a single device or an array of devices. The single device or array of devices can be enclosed by covers or hold in containers.

In one embodiment of the proposed invention the physical or chemical properties (dielectric constant, temperature, of the substrate can be modified dynamically.

In one embodiment of the proposed invention the substrate can contain MR visible markers/sensors used as concentration references, temperature standards, RF power deposition sensors or shift reagents.

In one embodiment of the proposed invention the substrate can be placed in a vacuum to change its electrodynamic properties.

In the embodiment of the proposed invention we suggest that the holes and apertures might be coated by a different material (e.g. gold or lead) for better attenuation of the ionizing radiation.

### REFERENCES AND ACKNOWLEDGEMENT

[1] US 5017872 A, Thomas K. Foo
[2] US 2011/0152670, Qing X. Young
[3] Garcia T. R. et al., "Dielectric collimators for linear collider beam delivery system", Proceedings of IPAC10, Kyoto Japan
[4] Vujayan T. et al., "Intense electron beam propagation by charge neutralization from a dielectric collimator", IEEE Transactions on plasma science, vol. 22, no. 2, April 1994
[5] US 5,191,289, Hayakawa et al.

The work leading to this invention has received funding from the European Union's Seventh Framework Programme (FP7/2007-2013).

### DESCRIPTION OF THE FIGURES

The figures provided herein represent examples of particular embodiments of the invention and are not intended to limit the scope of the invention. The figures are to be considered as providing a further description of possible and potentially preferred embodiments that enhance the technical support of one or more non-limiting embodiments. The figures illustrate basic schemes and implementations of preferred embodiments of the invention.
**Figure 1** shows the scheme of a usual magnetic resonance system. It is placed inside of a shielded room, has a magnet that generates a static magnetic field, a set of gradient coils for generating variable magnetic fields. The system includes RF coils for excitation of the nuclear magnetic spins. The object under investigation is placed on a table or a handling system that is driven into the MR system. Other components of the MR system include an RF source with RF amplifier, a gradient amplifier and an RF detector and digitizer. The whole system is controlled by a computer.
**Figure 2** shows the scheme of a hybrid SPECT/MR system. It is placed inside of a shielded room, has a magnet that generates a static magnetic field, a set of gradient coils for generating variable magnetic fields. The system includes RF coils for excitation of the nuclear magnetic spins. Between the RF coil and the gradient coils there are the components for the SPECT system including the collimator and the SPECT (or gamma camera) detectors. The object under investigation is placed on a table or a handling system that is driven into the MR system. Other components of the MR system include an RF source with RF amplifier, a gradient amplifier and an RF detector and digitizer. The whole system is controlled by a computer.

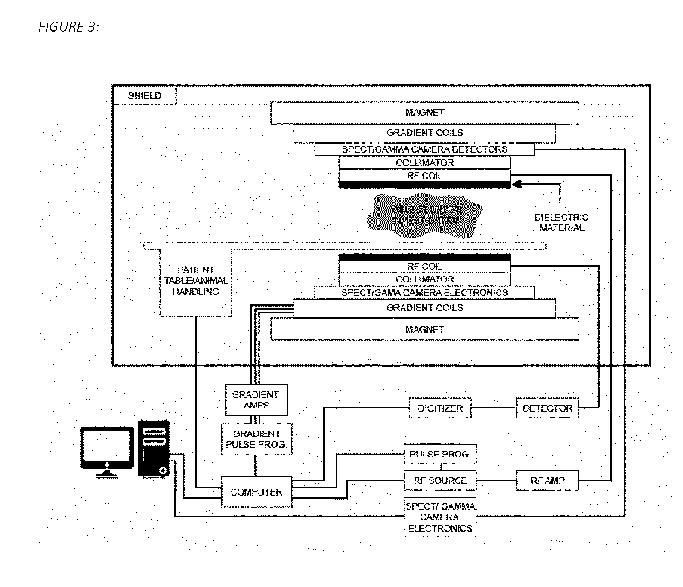

**Figure 3** shows the scheme of a hybrid SPECT/MR system with a dielectric material as proposed in this invention. It is placed inside of a shielded room, has a magnet that generates a static magnetic field, a set of gradient coils for generating variable magnetic fields. The system includes RF coils for excitation of the nuclear magnetic spins. Between the RF coil and the gradient coils there are the components for the SPECT system including the collimator and the SPECT (or gamma camera) detectors. Inside of the RF coil there is the dielectric material, whereas also the collimator can be manufactured from a dielectric material. The dielectric material has also a collimating function as proposed by this invention.
The object under investigation is placed on a table or a handling system that is driven into the MR system. Other components of the MR system include an RF source with RF amplifier, a gradient amplifier and an RF detector and digitizer. The whole system is controlled by a computer.

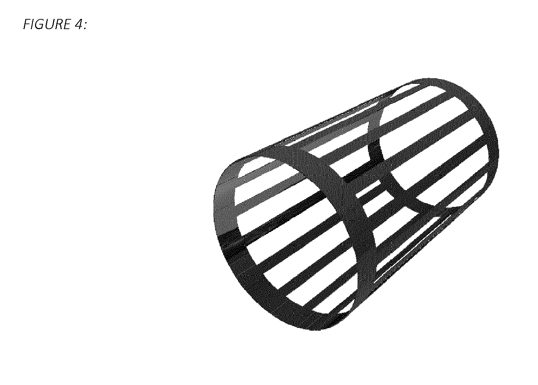

**Figure 4** shows an example of a volume RF coil that has a form of a volume birdcage. It has longitudinal rugs and end rings.

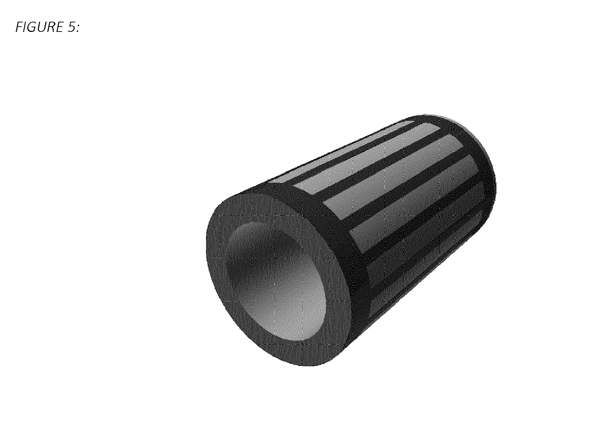

**Figure 5** shows an example of a volume RF coil that has a form of a volume birdcage. It has longitudinal rugs and end rings. At the inner side of the RF coil a substrate from a high dielectric material is placed that acts also as a collimator for ionizing radiation.

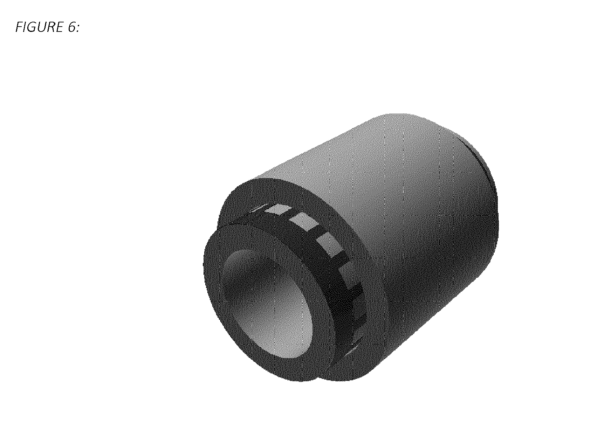

**Figure 6** shows an example of a volume RF coil that has a form of a volume birdcage. It has longitudinal rugs and end rings. At the inner side of the RF coil a substrate from a high dielectric material is placed that acts also as a collimator for ionizing radiation. At the outer side of the RF coil another part of the substrate is placed that acts as a collimator for ionizing radiation and can be also manufactured from a high dielectric material.

## Claims

1. A device that is either connected to an existing magnetic resonance system or is a part of a dedicated system for the purpose of hybrid application of single photon emission computed tomography and magnetic resonance imaging, comprising:
an RF coil which generates B₁ magnetic field;
a substrate from a high dielectric material that acts as a collimator of ionizing radiation and couples the RF field produced by the RF coil with the sample; and
a space inside of the substrate and the RF coil for accommodating an object under investigation.

2. A device according to claim 1, wherein the RF coil can be a volume or a surface RF coil or an array of RF coils.

3. A device according to claim 1, wherein the substrate is manufactured from at least one homogeneous material.

4. A device according to claim 1, wherein the substrate is manufactured from at least one material that presents a continuously changing physical parameter (e.g. density or permittivity) throughout the volume of the material

5. A device according to claim 1, wherein the substrate is a single unit or an array of units that can be enclosed by covers or hold in containers.

6. A device according to claim 1, wherein the physical or chemical properties of the substrate can be modified dynamically (temperature, dielectric constant).

7. A device according to claim 1, wherein the substrate is placed at least from one side of the RF coil.

8. A device according to claim 1, wherein at least one part of the substrate can act as an RF shield for the RF coil.

9. A device according to claim 1, wherein the substrate can contain MR visible markers/sensors used as concentration references, temperature standards, RF power deposition sensors or shift reagents.

10. A device according to claim 1, wherein the collimation performance can be further improved by coating or shielding of the holes and apertures with a different radiopaque material.

11. A device according to claim 1, wherein the substrate is composed of at least one material that has high dielectric constant.

12. A method of using a high dielectric constant material for a hybrid MRI/SPECT system for homogenizing the RF transmit field of an RF coil.

13. A method of using a high density material for a hybrid MRI/SPECT system for collimation and attenuation of the ionizing radiation.
